# EUROPÄISCHE PATENTSCHRIFT

(11) **EP 0 973 541 B1**
(45) Veröffentlichungstag und Bekanntmachung des Hinweises auf die Patenterteilung: **29.11.2006**
(21) Anmeldenummer: 98919254.7
(22) Anmeldetag: 09.04.1998
(51) Int. Cl.: A61K 38/16, G01N 33/574, C07K 14/705, A61P 35/00

(54) **VERWENDUNG VON HISTONEN ZUR HERSTELLUNG VON ARZNEIMITTELN**
USE OF HISTONS FOR THE PRODUCTION OF MEDICAMENTS
UTILISATION DES HISTONS POUR LA FABRICATION DES MEDICAMENTS

(30) Priorität: 11.04.1997 DE 19715149
(43) Veröffentlichungstag der Anmeldung: 26.01.2000
(73) Patentinhaber: SYMBIOTEC Gesellschaft zur Forschung und Entwicklung auf dem Gebiet der Biotechnologie GmbH, D-35745 Herborn-Hörbach (DE)
(72) Erfinder: ZEPPEZAUER, Michael, D-66133 Scheidt (DE); LEINENBACH, Hans-Peter, D-66636 Tholey (DE); CLASS, Reiner, Drexel Hill, PA 19026 (US); FASSBENDER, Cordula, D-50823 Köln (DE)
(74) Vertreter: Pätzold, Herbert
(86) Internationale Anmeldenummer: PCT/EP1998/002112
(87) Internationale Veröffentlichungsnummer: WO 1998/046252

(56) Entgegenhaltungen:
- EP-A- 0 392 315
- EP-A- 0 438 756
- WO-A-93/21943
- DE-A- 3 737 274
- R. CLASS ET AL.: "HISTONE H1 SUPPRESSES TUMOR GROWTH OF LEUKEMIA CELLS IN VITRO, EX VIVO AND IN ANIMAL MODEL SUGGESTING EXTRACELLULAR FUNCTIONS OF HISTONES." AMERICAN JOURNAL OF CLINICAL ONCOLOGY (CANCER CLINICAL TRIALS), Bd. 19, Nr. 5, Oktober 1996, Seiten 522-531, XP002073894 NEW YORK, N.Y., US in der Anmeldung erwähnt

## Beschreibung

Die Erfindung betrifft die Verwendung von Histonen oder deren therapeutisch wirksamen Abschnitten zur Herstellung von Arzneimitteln gemäß des Anspruches 1.

Nach Reiner Class et al. in Am. J. Clin. Oncol. (CCT) 19 (5) 1996, S. 552-531 ist die Wirkung von Histon H1 auf Krebszellen dadurch erklärt, daß Krebszellen, welche von Histon H1 abgetötet werden, in ihrer Zellmembran ein sogenanntes Rezeptorprotein besitzen, welche das verabreichte Histon H1 binden kann. Das Rezeptorprotein wurde durch Affinitätschromatographie an immobilisiertem Histon H1 isoliert. Nach Ergebnissen der SDS-PAGE Elektrophoresetechnik besitzt das Rezeptorprotein eine phoretische Beweglichkeit, die einer Molmasse im Bereich von 33000 Da (33 kDa) ± 2000 Da entspricht.

Die Bindung von H1 mit dem membranständigen Rezeptorprotein führt zu einer Zerstörung der Membranintegrität, so daß lösliche Zellbestandteile, wie gelöste Proteine, lonen und andere Stoffe unkontrolliert aus der Krebszelle nach außen entweichen oder anderer Stoffe in die Zelle einströmen können und die Zelle dadurch abstirbt.

Die Erfindung geht von der Überlegung aus, daß Tumorzellen unterschiedlichen histologischen Ursprunges, z.B. Leukämien, Lymphome, Sarkome, Carcinome, Melanome u.a. besondere individuelle Eigenschaften besitzen und daß bei näherer Kenntnis dieser individuellen zellinienspezifischen Eigenschaften diese einen Ansatz für eine neue wirksame Therapie bieten können. Es konnte gezeigt werden, daß das Rezeptor überraschenderweise nicht charakteristisch ist für einen oder mehrere bestimmte Zelltypen, sondern eine individuelle zellinientypische Eigenschaft von Krebszellen darstellt, die einer gezielten Therapie zugänglich gemacht werden konnte.

Die Erfindung hat sich damit zum Ziel gesetzt, nähere Kenntnisse über den Rezeptor zu erhalten, die eine neue Lehre zur therapeutischen und prophylaktischen Behandlung bzw. Unterdrückung von solchen individuellen Krebszellen mit derartigen Rezeptoren vermitteln.

Die zielsetzung umfaßt damit auch eine Diagnose zur Erkennung von Krebserkrankungen, die als individuelle Eigenschaft solche Rezeptoren enthalten, so daß der Erfolg der neuen Lehre zur therapeutischen Behandlung solcher individueller Krebszellen auch vorherbestimmbar ist und damit gezielt zur Anwendung gebracht werden kann. Dies bedeutet, daß das Krankheitsbild nach Maßgabe des Erfolges einer Behandlung mit dem histonhaltigen Therapeutikum auf eine neue Weise klassifizierbar ist. Sobald gesicherte Erkenntnisse vorliegen, die die Neigung von bestimmten Menschen zu derartigen individuellen Krebszellen vorhersehen lassen, ist das histonhaltige Therapeutikum auch prophylaktisch anwendbar. Das wird vor allem dann der Fall sein, wenn bei diesen Menschen der natürliche Histonspiegel im Blut als zu niedrig erkannt ist.

Der Erfindung liegt die Erkenntnis zugrunde, daß der vorstehende Rezeptor in der Membran von individuellen Krebszellen, insbesondere Krebszellen des blutbildenden Systems, wie Leukämien, Lymphome, Myelome, verschiedene Histone und/oder deren therapeutisch wirksame Teile enthält die eine Bindung oder Vernetzung mit zugeführten Histonen oder deren wirksamen Abschnitten eingehen, zur diagnose können auch geeignete Antikörper verwendet werden.

Die Erfindung ist durch die kennzeichnenden Merkmale des Anspruchs 1 gelöst. Vorteilhafte Ausführungen ergeben sich aus den Merkmalen der Unteransprüche.

Es wurde zunächst der vorstehend erwähnte Rezeptor in der Membran von individuellen Krebszellen charakterisiert.

Eine massenspektrometrische Analyse mittels der MALDI-MS Technik (matrix-assisted laser desorption/ionization mass spectrometry) ergab, daß die elektrophoretisch einheitliche Proteinbande drei Proteine mit den Molmassen 11,2; 13,7 und 15 kDa enthielt.

Im einzelnen ergaben sich folgende Mittelwerte:

| | | | |
|---|---|---|---|
| 11175 | ± | 40 Da | |
| 13730 | ± | 40 Da | und |
| 15035 | ± | 80 Da | |

Die Mittelwerte berechnen sich aus einfach- und doppelt geladenen Molekülionensignalen und vier bis sechs Summenspektren, die von der untersuchten Probe aufgenommen werden.

Im Vergleich zu den vorstehenden drei Molmassen der Polypeptide des Rezeptors weisen die nachstehenden humanen Histonsequenzen folgende Molmassen auf:

| | | |
|---|---|---|
| H4 | 11282 Da | |
| H2B | 13774 Da | |
| H3 | 15324 Da | und |
| H2A | 14004 Da | |

Die Sequenzanalyse mittels automatisiertem Edman-Abbau identifizierte eine Peptidsequenz, welche dem Abschnitt 23-59 des humanen Histons H4 entspricht, sich aber in den Positionen 23 und 35 unterscheidet, was einer Homologie von 97,2% bezogen auf die bekannte Sequenz des Histons H4 in gesunden Zellen entspricht.

Diese Befunde lassen den überraschenden Schluß zu, daß der besagte Rezeptor aus Proteinen besteht oder solche Proteine enhält, die den Histonen H4, H2B und H3 zugeordnet werden können oder ihnen sehr ähnlich sind. Ihr Vorkommen in der Membran von individuellen Krebszellen ist mit großer Wahrscheinlichkeit auf die tumorbedingte Entartung zurückzuführen, desgleichen ihre mengenmäßige Zusammensetzung und möglichen Strukturunterschiede zu den im Nucleosom befindlichen Histonen.

Hiermit lassen sich die auf der Zehmembran von Krebszellen in vergleichsweiser hoher Konzentration vorkommenden Histone der Klassen H2A, H2B, H3 und/oder H4 (die sog. Core-Histone) durch äußere Zugabe von Histonen, insbesondere Histon H1 oder therapeutisch wirksamen Sequenzteilen derselben zu größeren Aggregaten vernetzen, wodurch die Membranintegrität zerstört wird und die Krebszellen abgetötet werden. Bei den Histonen kann es sich um natürliche Histone, z.B. aus bovinem Material oder auch um rekombinante Histone handeln. Es kann sich auch um kovalent modifizierte Histone oder Histonabschnitte oder funktionell und strukturell ähnliche Proteine handeln. Als geeignete kovalente Modifikationen können z.B. Derivate mit Polyoxyethylenketten ("PEGylierung") dienen. Funktionell oder strukturell ähnliche Proteine sind z. B. Protamine oder histonähnliche Proteine aus Prokaryonten oder Arche.

Es ist bekannt, daß die Histon H1-Moleküle die zu den sog. Nucleosomen organisierten Aggregate der Core-Histone weiter verknüpfen können, wodurch die kondensierte Form des Chromatins entsteht. Dazu sind weitgehend intakte H1-Moleküle notwendig, welche einen kompakt gefalteten Mittelteil (den "globulären" Teil) und die angeschlossenen flexiblen, N- und C-terminalen Domänen aufweisen.

Aus dieser Erkenntnis ließ sich eine Methode ableiten, den zytotoxisch wirksamen Bereich des Histon H1 Moleküls zu ermitteln, der eine Vernetzung oder Bindung mit dem Core-Histonen in Zellmembranen von individuellen Krebszellen weitgehend sicherstellt.

Hierzu wurden in Zytotoxizitätsstudien als wirksame Substanzen teils das rekombinant gewonnene Histon H5 aus Hühnererythrozyten eingesetzt, welches eine erythrozytenspezifische Variante des H1 darstellt. Teils wurden Fragmente dieses Histons eingesetzt, bei welchen entweder beide der terminalen Domänen stark gekürzt wurden, oder die C-terminale Domäne sukzessive verkürzt wurde. Diese Fragmente und ihre gentechnische Herstellung wurden von Gerchman et al in Protein Expression & Purification, 5 (1994), Seiten 242-251 beschrieben.

Die nachstehende Abbildung zeigt die verwendeten H5 Fragmente und ihren strukturellen Aufbauend zwar das vollständige Molekül H5, im wesentlichen nur seinen globularen Abschnitt (GH5), den globularen Abschnitt in Verbindung mit der N-terminalen Domäne (NGH5), den globulären Abschnitt in Verbindung mit der N-terminalen Domäne und demC-terminalen Abschnitt 98-131 (NGC1H5) und schließlich den globulären Abschnitt in Verbindung mit der N-terminalen Domäne und dem C-terminalen Abschnitt 98-151 (NGC2H5). Alle eingesetzten Proteine und Kulturmedien waren auf Endotoxinfreiheit getestet.

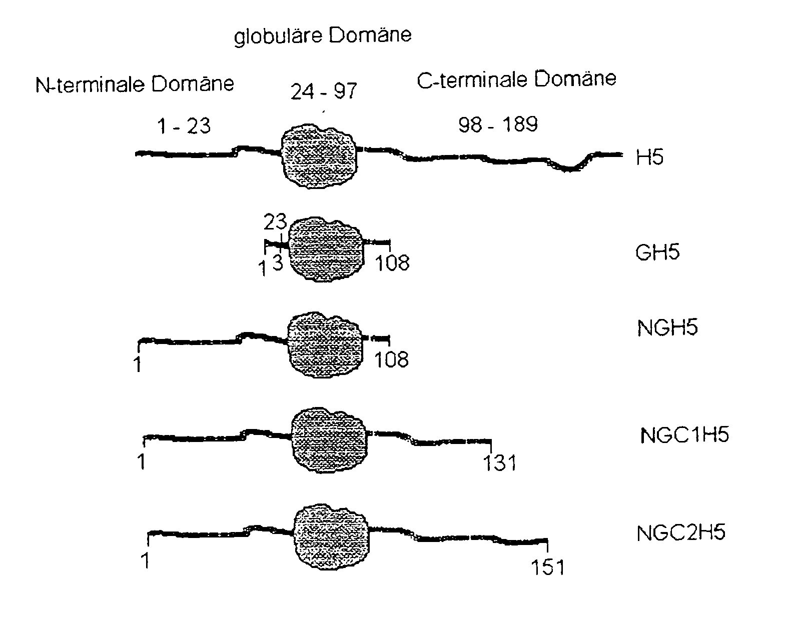

Die vorstehende schematische Darstellung der 3 strukturellen Domänen des Moleküls Histon H5, GH5, NGH5, NGC1H5 und NGC2H5 bezeichnen rekombinant gewonnene Histonfragmente.

Zur Untersuchung möglicher Struktur-Funktionsbeziehungen der Wirkung von H1 aus Kalbsthymus auf entartete Zellen wurden Vergleichsexperimente mit den zur Verfügung stehenden, rekombinant gewonnenen Histonteilsequenzen aus H5 gemacht. Es wurden die primären Leukämiezellen einer Patientin mit AML, die Leukämiezellen der Linie IM9 und die Lymphomzellen der Zellinie QH77 getestet. In allen Ansätzen wirkte H1 in einer Konzentration von 250 µg/ml zytotoxisch. Die längeren Teilsequenzen NGC1 H5 und NGC2H5 hatten im Fall der AML und der Lymphomzellen zu H1 vergleichbare, bei den Zellen der Zellinie IM9 sogar stärkere Wirkung als Histon H1. Der globuläre Teil GH5 und die Teilsequenz NGH5 provozierten bei allen Zelltypen die geringsten, wachstumshemmenden Effekte. Die Teilsequenzen wurden jeweils in zu H1 äquimolaren Mengen eingesetzt. Die Ergebnisse sind nachstehend in den Beispielen 1 bis 5 zusammengefaßt.

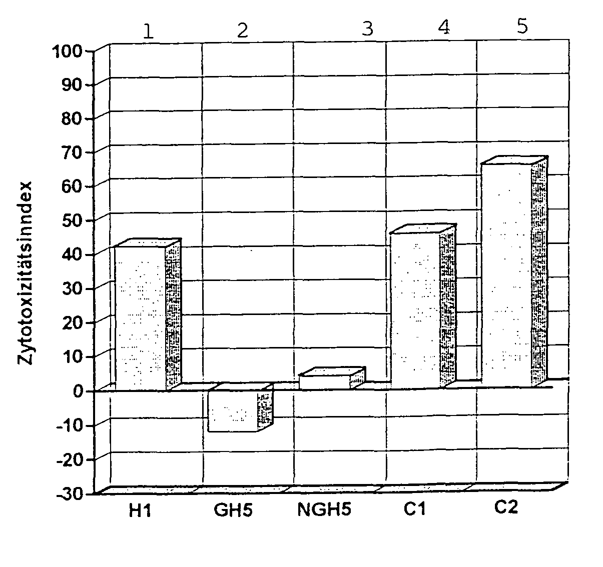

Das vorstehende Diagramm zeigt die Zytotoxizität von Histon H1 und Histonteilsequenzen für Primäre Leukämiezellen einer Patientin mit AML (akute myelomische Leukämie).

In den Beispielen 1 bis 5 wurden 1 x 10⁶ Zellen/ml für 48h unter Standardbedingungen wie folgt inkubiert:
**H1:** Histon H1; rekombinante Teilsequenzen von Histon H5: **GH5:** Teilsequenz GH5; **NGH5:** Teilsequenz NGH5; **C1:** Teilsequenz NGC1 H5; **C2:** Teilsequenz NGC2H5. H1, bzw die Histonteilsequenzen wurden 12 µM eingesetzt. Die Vitalität der Zellen wurde mit der MTT-Methode bestimmt. Dargestellt sind die Mittelwerte aus je 4 Parallelexperimenten.

Die nachstehenden Beispiele 6 bis 10 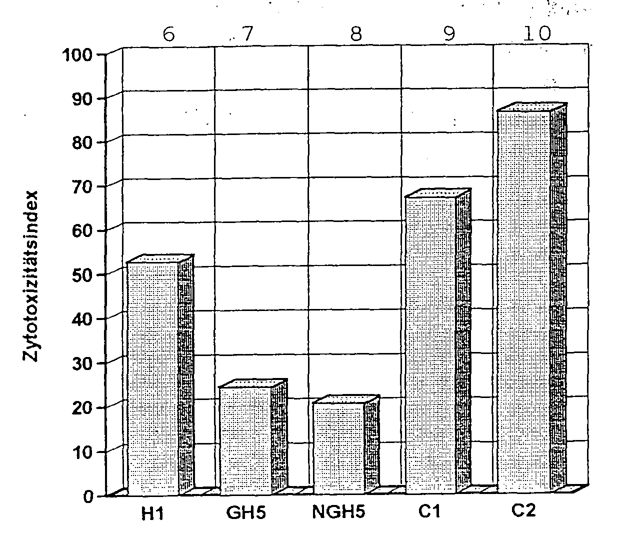 zeigen die Zytotoxizität von Histon H1 und Histonteilsequenzen für die Zellen einer myelomischen Leukämie der Zellinie IM9.

Weiterhin zeigen die nachstehenden Beispiele 11 bis 15 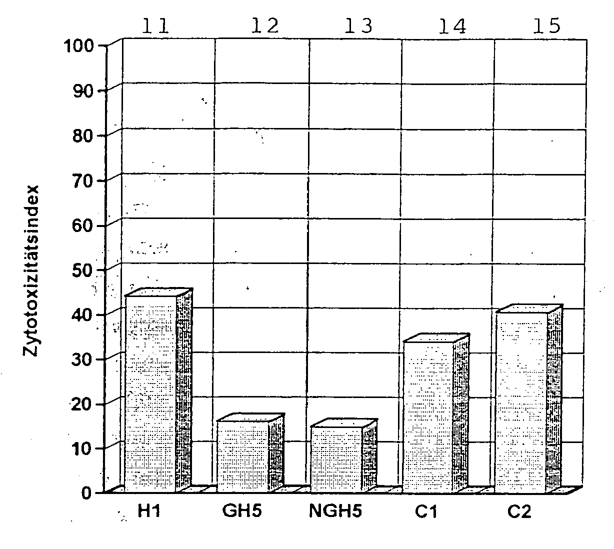 die Zytotoxizität von Histon H1 und Histonteilsequenzen für die Zellen eines Lymphoms der Zellinie OH77.

In den Beispielen 6 bis 15 wurden 5 x 10⁴ Zellen/ml für 48h unter Standardbedingungen wie folgt inkubiert:
**H1:** Histon H1; rekombinante Teilsequenzen von Histon H5: **GH5:** Teilsequenz GH5; **NGH5:** Teilsequenz NGH5; **C1:** Teilsequenz NGC1H5; **C2:** Teilsequenz NGC2H5. H1, bzw. die Histonteilsequenzen wurden 12 µM eingesetzt. Die Vitalität der Zellen wurde mit der MTT-Methode bestimmt. Dargestellt sind die Mittelwerte aus je 4 Parallelexperimenten.

Aus den Beispielen geht hervor, daß die globuläre Domäne des H1 (oder H5) Moleküls GH5 eine nur geringe oder keine zytotoxische Wirkung aufweist (Cytotoxizitätsindex unter 20). Ähnliches gilt für das Fragment NGH5, bei welchem die C-terminale Domäne fast völlig fehlt. Wenn diese Domäne mit wenigstens den Resten 108 - 131 vertreten ist und die N terminale Domäne intakt ist (NGC1 H5), dann liegt ein Fragment vor, dessen Wirksamkeit die des intakten Histonmoleküls erreichen oder übertreffen kann (Beispiele 4 und 9). Ein Gleiches gilt für das Fragment NGc2H5 in welchem nur noch 38 Aminosäuren der C-terminalen Domäne fehlen.

Ähnliche Beziehungen gelten entsprechend für Fragmente mit verkürzter N-terminaler Domäne und intakter C-Domäne.

Es liegt nahe, statt Histon H5 humane Subtypen von H1, z.B. insbesondere H1.1; H1.2; H1.3; H1.4 und H1.O oder deren Teile erfindungsgemäß einzusetzen.

### Beispiele 16 bis 18

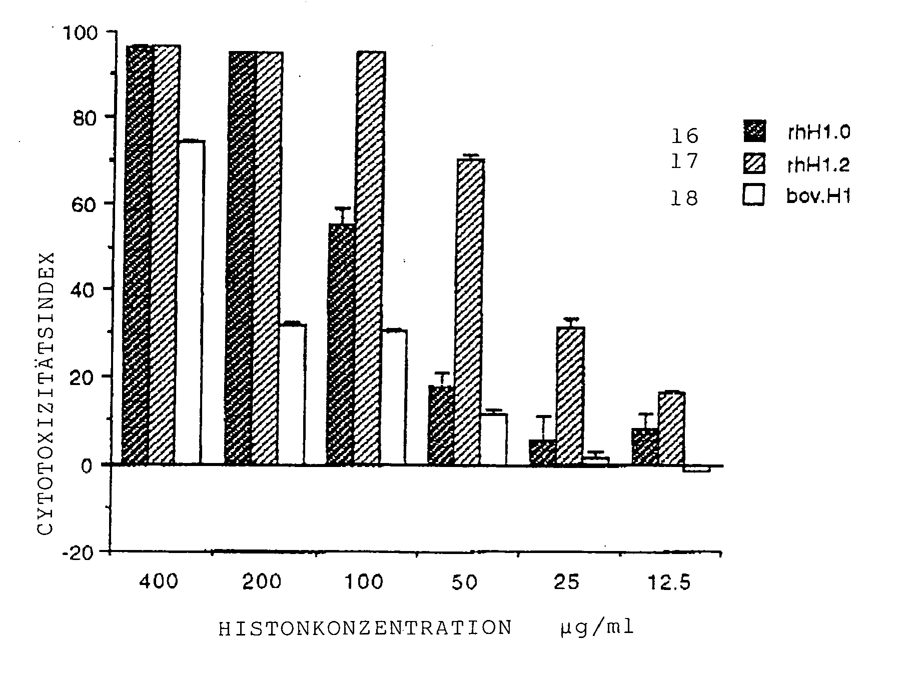
100
80
60
40
20
0
-20
CYTOTOXIZITÄTSINDEX
16
17
18
rhH1.0
rhH1.2
bov.H1
400
200
100
50
25
12.5
HISTONKONZENTRATION   µg/ml

Die Beispiele 16 und 17 zeigen die Wirksamkeit der rekombinant (rh) gewonnen humanen Subtypen H1.0 und H1.2 im Vergleich zu den aus Kalbsthymus (bov) gewonnenen Histonen H1 auf Zellen eines Burkitt-Lymphoms der Zellinie Daudi (Beispiel 18).

Die nachstehende Tabelle 2 zeigt die genannten humanen H1-Subtypen mit ihren N- und C-terminalen Domänen jeweils in Verbindung mit der hier nicht genannten globulären Domäne zwischen den N- und C-terminalen Domänen sowie den notwendigen N- und C-terminalen Abschnitten bei denen in Analogie zu den vorstehenden Ergebnissen mit dem Histon H5 vom Huhn zytotoxische Wirkung gegenüber Krebszellen mit membranständigen Core-Histonrezeptoren zu erwarten sind.

**TABELLE 2**

| N-termin. | C-termin. | notwendige. | notwendige. |
|---|---|---|---|
| Domäne | Domäne | N-Domäne | C-Domäne |
| H 1.1. 1-40 | 118-214 | ab 16;20-26 | 118-138 |
| H 1.2. 1-38 | 105-212 | ab 16;20-26 | 105-125 |
| H 1.3. 1-39 | 117-220 | ab 16;20-26 | 117-137 |
| H 1.4. 1-38 | 116-218 | ab 16;20-26 | 116-136 |
| H 1.0. 1-25 | 99-193 | ab 11;16-20 | 99-119 |

In der hier vorgenommenen Einteilung wurden als C- und N-terminale Bereiche diejenigen definiert, welche in ihren Sequenzen Prolin (P) enthalten, weil Prolin die Ausbildung von Helices verhindert, da es eine Streckung der Polypeptid-Kette bewirkt.

Die Grenzen für die Domänen werden in der Literatur unterschiedlich angegeben. Beim H5 des Huhns zählen manche Autoren den N-Terminus von 1-18 und die globuläre Domäne 19-108, während Gerchman et al. in der vorstehenden Literaturstelle diese als die Sequenz 24-96 definieren.

Zusammenfassend ergibt sich, daß die Proteine des individuellen Krebszellenrezeptors nach derzeitigen Erkenntnissen ein Aggregat aus den Core-Histonen H2A, H2B, H3 und/oder H4 darstellt oder solche Histone oder coreähnliche Histone enhält. Ob auch DNA an der Membranoberfläche vorkommt kann derzeit nicht mit Bestimmtheit gesagt werden. Ausgeschlossen ist es nicht.

Es ist ebenfalls nicht ausgeschlossen, daß infolge der neoplastischen Entartung in der Krebszelle Protamine synthetisiert werden können und statt der Core-Histone oder gemeinsam mit ihnen in der Zellmembran lokalisiert werden, die mit Histonen oder histonähnlichen Proteinen vernetzbar oder bindbar sind.

Die dem Kern des Nukleosoms angehörenden Histone (Core-Histone) sind durch nichtkovalente Kräfte stark aneinander gebunden und täuschen somit ein höheres Molekulargewicht vor. Auch auf der Zellmembran, in die sie durch eine tumorbedingte Fehlregulation gelangen, sind sie miteinander assoziiert.

Der Wirkungsmechanismus des erfindungsgemäßen Therapeutikums beruht auf einer weiteren Vernetzung der membranständigen Histone durch (a) von außen angebotene Histone, insbesondere H1 und deren vernetzbaren Teile, (b) durch wirksame kovalent modifizierte Histone oder (c) durch funktionell und/oder strukturell histonähnliche Proteine. Durch die Vernetzung oder Bindung entstehen in der Zellmembran größere Aggregate, die Porencharakter besitzen oder zu Porenbildung in der Membran Anlaß geben oder auch mechanische Deformationen von Zellmembrankomponenten, die mit den Strukturen des Zytoskeletts assoziiert sind, hervorrufen, was zu einer Zerstörung der Membranintegrität und letztlich zum Zelltod führt. Die Beteiligung von Signalweiterleitungsmechanismen und apoptotischen Progressen ist möglich, ist aber nicht der maßgebliche Prozeß, der zum Zelltod führt.

Die Wirksamkeit des erfindungsgemäßen Therapeutikums ist in den nachfolgenden Photografien A bis D verdeutlicht:

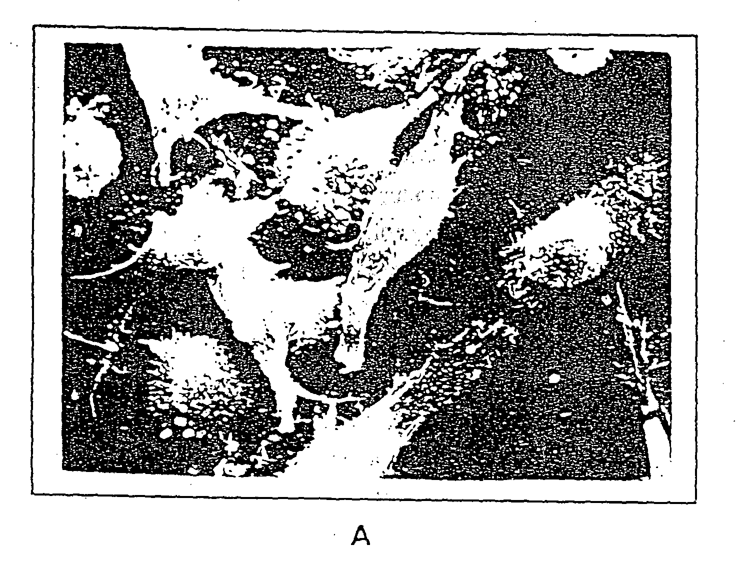
A

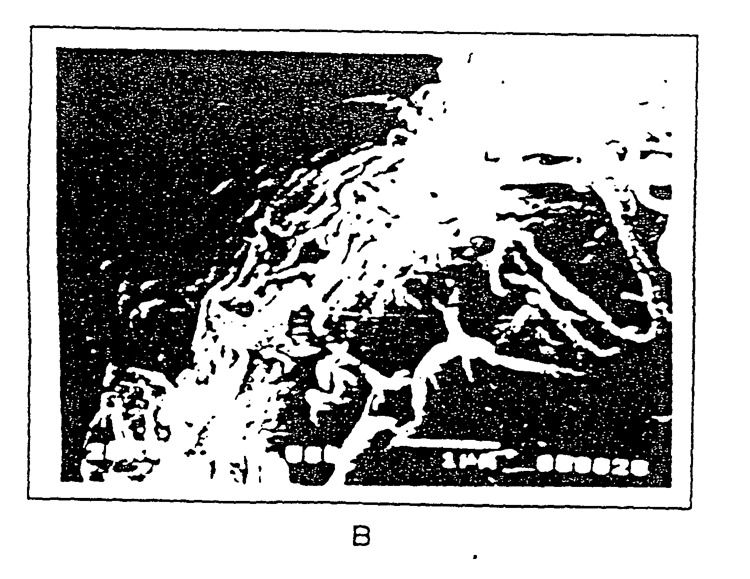
[?][?][?]   1[?][?] [?][?][?][?]2[?]

B

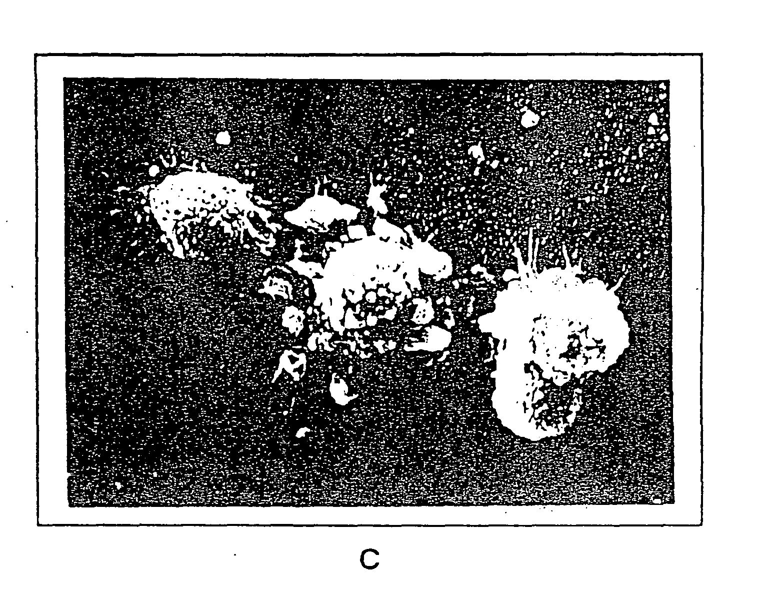
C

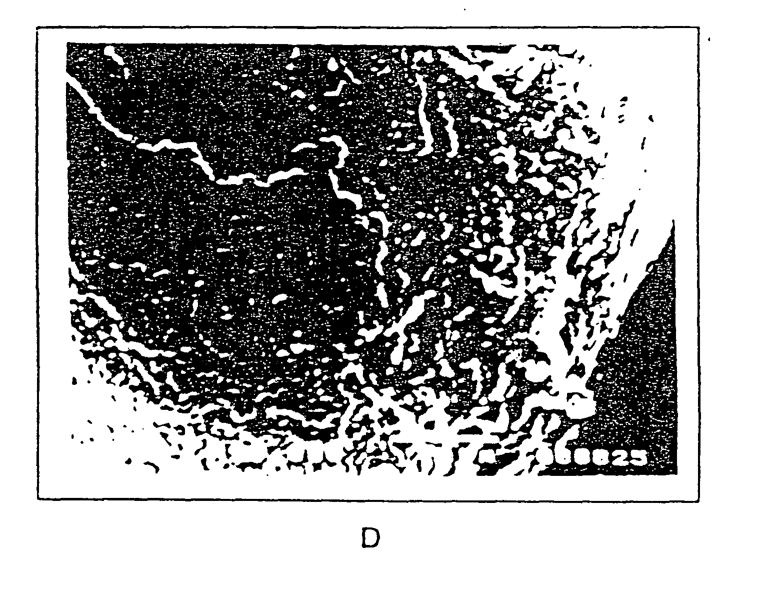
[?]60025
D

Die Fotografie A zeigt unbehandelte Zellen der Lymphomzellinie OH77 in 2000-facher Vergrößerung, die Fotografie B zeigt einen Ausschnitt aus A in 20.000-facher Vergrößerung. Hierdurch wird die typische Oberflächenstruktur einer intakten lymphatischen Tumorzelle verdeutlicht. Die Photografie D zeigt in 20.000-facher Vergrößerung eine drastische Veränderung dieses Zellausschnittes nach einer 12 Std. Einwirkzeit mit 200µg/ml Histon H1. Es zeigt sich hier, das die Zellen zu einer kugeligen Gestalt kontrahieren, bis sie nach einer Behandlungszeit mit Histon H1 nach 24 Std. disintegrieren wie die Photografie C zeigt.

Verwendet wurde das zugesetzte reine Histon H1 in der oben angegebenen Konzentration jeweils in einem normalen Medium für die Kultivierung von Zellen, wie es in der eingangs genannten Literaturstelle beschrieben ist.

Die individuellen Krebszellen mit den besagten Rezeptorproteinen in den Membranen dieser Krebszellen können diagnostiziert werden. Zur Diagnose werden Histone, insbesondere H1 oder deren wirksamen Teile oder geeignete Antikörper verwendet, die an das Reszeptorprotein binden, wodurch erstmals eine Klassifizierung von individuellen Krebszellen ermöglicht ist, die die besagten Rezeptoren aufweisen. Die hierbei zu verwendenden Diagnosetechniken gehören zum Stand der Technik. Zum Stand der Technik gehört auch die Auffindung geeigneter Antikörper, welche die Rezeptorproteine in der Membran der Krebszellen oder die durch die Aggregation entstehenden Strukturen erkennen.

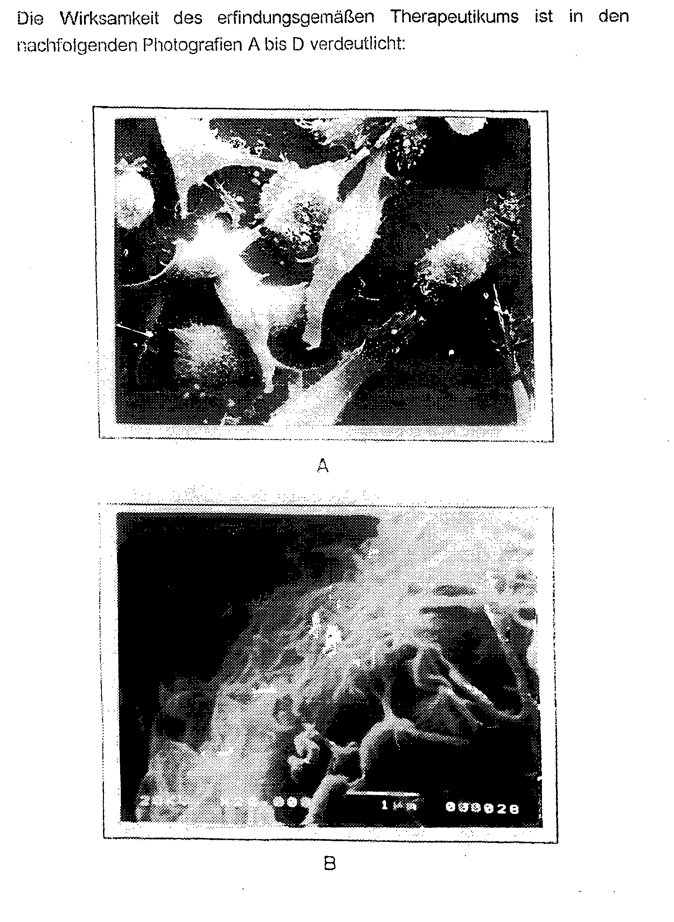

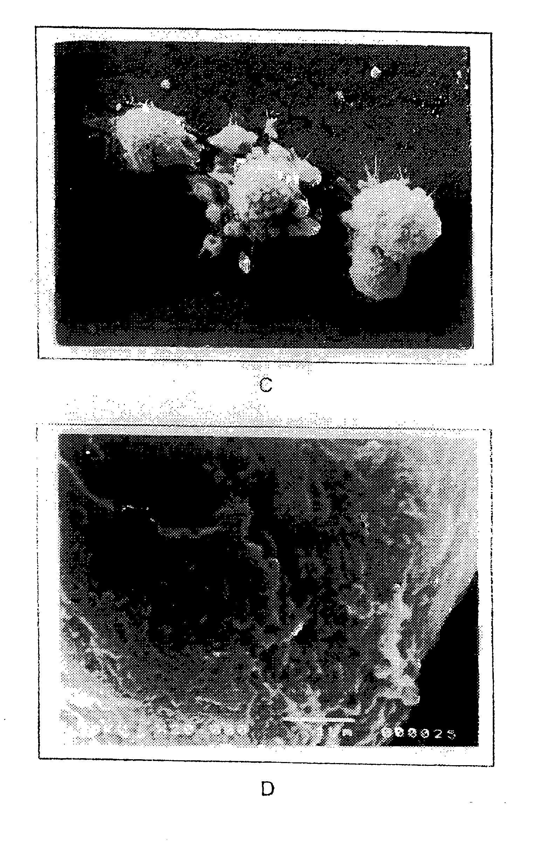

## Patentansprüche

1. Verwendung von Histonen oder deren therapeutisch wirksamen Abschnitten zur Herstellung eines Arzneimittels zur Therapie von Krebserkrankungen welche **dadurch** charakterisiert sind, daß die Membranen der Krebszellen Rezeptoren enthalten, die aus den Histonen H2A, H2B, H3 und/oder H4 bestehen.

2. Verwendung nach Anspruch 1, **dadurch gekennzeichnet, daß** das Arzneimittel Histon H1 oder Histon H5 enthält.

3. Verwendung nach Anspruch 2 **dadurch gekennzeichnet, daß** das Arzneimittel wenigstens einen humanen Histon H1-Subtyp enthält.

4. Verwendung nach Anspruch 2, **dadurch gekennzeichnet, daß** das Arzneimittel wenigstens einen rekombinanten Histon H1-Subtyp enthält.

## Claims

1. Use of histones or therapeutically active parts thereof for the manufacturing of a medication for the therapy of cancerous diseases wherein the cancer cell membranes contain receptors that are composed of histones H2A, H2B, H3 and/or H4.

2. Use according to claim 1, wherein said medication contains histone H1 or histone H5.

3. Use according to claim 2, wherein said medication contains at least one human histone H1 subtype.

4. Use according to claim 2, wherein said medication contains at least one recombinant histone H1 subtype.

## Revendications

1. Utilisation d'histones ou de leurs fragments actifs sur le plan thérapeutique pour la fabrication d'un médicament pour la thérapie de maladies cancéreuses, lesquelles sont **caractérisées en ce que** les membranes des cellules cancéreuses contiennent des récepteurs qui comprennent les histones H2A, H2B, H3 et/ou H4.

2. Utilisation selon la revendication 1, **caractérisée en ce que** le médicament contient l'histone H1 ou l'histone H5.

3. Utilisation selon la revendication 2, **caractérisée en ce que** le médicament contient au moins un sous-type humain de l'histone H1.

4. Utilisation selon la revendication 2, **caractérisée en ce que** le médicament contient au moins un sous-type recombiné de l'histone H1.
